# EUROPÄISCHE PATENTANMELDUNG

(11) **EP 0 824 913 A2**
(43) Veröffentlichungstag der Anmeldung: **25.02.1998**
(21) Anmeldenummer: 97113162.8
(22) Anmeldetag: 30.07.1997
(51) Int. Cl.: A61K 7/42

(54) **Verwendung von Campherderivaten zur Erzielung oder Erhöhung der Löslichkeit von Triazinderivaten in Ölkomponenten**

(30) Priorität: 16.08.1996 DE 19633012
(71) Anmelder: Beiersdorf Aktiengesellschaft, 20245 Hamburg (DE)
(72) Erfinder: Gers-Barlag, Heinrich, Dr., 25495 Kummerfeld (DE); Kröpke, Rainer, 22527 Hamburg (DE)

(57) **Zusammenfassung**

Verwendung von Campherderivaten zur Erzielung oder der Erhöhung der Löslichkeit von 4,4',4''-(1,3,5-Triazin-2,4,6-triyltriimino)-tris-benzoesäure-tris(2-ethylhexylester) in
(a) entweder einer isolierten Ölkomponente oder
(b) in mindestens einer Ölphase eines dispersen Zwei- oder Mehrphasensystem welches zusätzlich eine oder mehrere Wasserphasen enthalten kann.

## Beschreibung

Die vorliegende Erfindung betrifft kosmetische und dermatologische Lichtschutzzubereitungen, insbesondere hautpflegende kosmetische und dermatologische Lichtschutzzubereitungen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutze gegen UVB-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altem läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, z.B. Hydroxylradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Ein vorteilhafter UVB-Filter ist der 4,4',4''-(1,3,5-Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5-triazin.

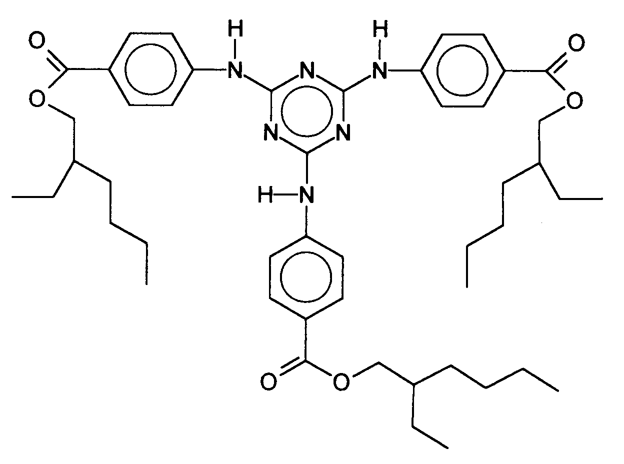

Diese UVB-Filtersubstanz wird von der BASF Aktiengesellschaft unter der Warenbezeichnung UVINUL® T 150 vertrieben und zeichnet sich durch gute UV-Absorptionseigenschaften aus.

Der Hauptnachteil dieses UVB-Filters ist die schlechte Löslichkeit in Lipiden. Bekannte Lösungsmittel für diesen UVB-Filter können maximal ca. 15 Gew.-% dieses Filters lösen, entsprechend etwa 1 - 1,5 Gew.-% gelöster, und damit aktiver, UV-Filtersubstanz.

Weiterhin sind bekannte und gebräuchliche Lichtschutzfiltersubstanzen der 4-Methylbenzylidencampher, welcher sich durch die Struktur 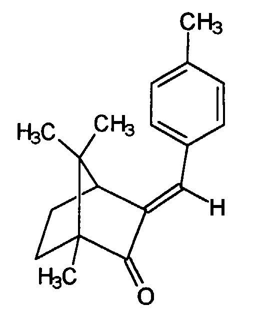 auszeichnet und von Merck unter der Marke Eusolex® 6300 verkauft wird, sowie der Benzylidencampher, welcher sich durch die Struktur 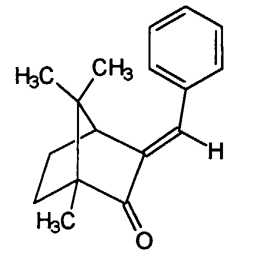 auszeichnet und von der Gesellschaft Induchem unter der Marke Unisol® S22 verkauft wird.

Dennoch bestand der Nachteil des Standes der Technik, daß in der Regel entweder nur vergleichsweise niedrige Lichtschutzfaktoren erreicht werden konnten, oder daß die Lichtschutzfilter nicht die genügende UV-Stabilität aufwiesen oder nicht genügende physiologische Verträglichkeit aufwiesen oder nicht genügend hohe Löslichkeit oder Dispergierbarkeit in kosmetischen oder dermatologischen Zubereitungen aufwiesen oder auch sonstige Inkompatibilitäten mit kosmetischen oder dermatologischen Zubereitungen oder mehrere Nachteile zugleich.

Wenigstens einigen, wenn nicht allen diesen Nachteilen abzuhelfen, war eine der Aufgaben der vorliegenden Erfindung.

Es war indes überraschend und für den Fachmann nicht vorauszusehen, daß die Verwendung von Campherderivaten der allgemeinen Struktur 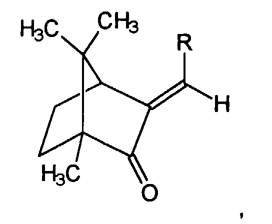 wobei R einen mit bis zu fünf Alkylgruppen substituierten oder unsubstituierten Arylrest darstellt, zur Erzielung oder der Erhöhung der Löslichkeit von 4,4',4''-(1,3,5-Triazin-2,4,6-triyltriimino)-tris-benzoesäure-tris(2-ethylhexylester) in
(a) entweder einer isolierten Ölkomponente oder
(b) in mindestens einer Ölphase eines dispersen Zwei- oder Mehrphasensystem welches zusätzlich eine oder mehrere Wasserphasen enthalten kann,
den Nachteilen des Standes der Technik abhilft.

Erfindungsgemäß bevorzugt wird das oder werden die Campherderivate gewählt aus der Gruppe Benzylidencampher, 4-Methylbenzylidencampher.

Bei erfindungsgemäßer Verwendung des oder der Campherderivate hat die relativ schweriösliche Komponente 4,4',4''-(1,3,5-Triazin-2,4,6-triyltriimino)-tris-benzoesäure-tris(2-ethylhexylester) eine bedeutend bessere Löslichkeit in
(a) entweder einer isolierten Ölkomponente oder
(b) in mindestens einer Ölphase eines dispersen Zwei- oder Mehrphasensystem welches zusätzlich eine oder mehrere Wasserphasen enthalten kann,
als in den Zubereitungen des Standes der Technik.

Es ist erfindungsgemäß bevorzugt, die dispersen Zwei- oder Mehrphasensystem welches zusätzlich eine oder mehrere Wasserphasen enthalten können in Form kosmetischer oder dermatologischer Emulsionen, beispielsweise vom Typ W/O, O/W, W/O/W oder O/W/O auszugestalten.

Selbst wenn als eine der Ölkomponenten, als die überwiegende Ölkomponente oder als die einzige Ölkomponente, sei es in isolierter Form oder in mindestens einer Ölphase eines dispersen Zwei- oder Mehrphasensystem welches zusätzlich eine oder mehrere Wasserphasen enthalten kann, der Dicaprylylether oder vergleichbare Substanzen gewählt wird oder werden, ist die Löslichkeit des 4,4',4''-(1,3,5-Triazin-2,4,6-triyltriimino)-tris-benzoesäure-tris(2-ethylhexylester) in den betreffenden Systemen erheblich erhöht.

Ferner sind erfindungsgemäß Lichtschutzzubereitungen erhältlich, welche höhere Stabilität, insbesondere Stabilität gegen Zersetzung unter dem Einfluß von Licht, ganz besonders UV-Licht, aufweisen, als der Stand der Technik hätte erwarten lassen. Weiterhin sind erfindungsgemäß besonders gut hautverträgliche Zubereitungen erhältlich.

Voraussetzung für die Verwendbarkeit der erfindungsgemäßen Wirkstoffkombinationen für die erfindungsgemäßen Zwecke ist natürlich die kosmetische bzw. dermatologische Unbedenklichkeit der zugrundeliegenden Substanzen.

Es ist erfindungsgemäß möglich die Einsatzmengen von 4,4',4''-(1,3,5-Triazin-2,4,6-triyltriimino)-tris-benzoesäure-tris(2-ethylhexylester) in kosmetischen oder dermatologischen Zubereitungen gegenüber dem Stande der Technik beispielsweise zu verdoppeln.

Ferner war erstaunlich, daß durch Zugabe erfindungsgemäßer Campherderivate, insbesondere durch Zugabe von Benzylidencampher und/oder 4-Methylbenzylidencampher eine Stabilisierung von Lösungen des 4,4',4''-(1,3,5-Triazin-2,4,6-triyltriimino)-tris-benzoesäure-tris(2-ethylhexylester) in
(a) entweder einer isolierten Ölkomponente oder
(b) in mindestens einer Ölphase eines dispersen Zwei- oder Mehrphasensystems, welches zusätzlich eine oder mehrere Wasserphasen enthalten kann,
bewirkt wird, da der4,4',4''-(1,3,5-Triazin-2,4,6-triyltriimino)-tris-benzoesäure-tris(2-ethylhexylester) normalerweise nicht nur schlechte Löslichkeit aufweist, sondern auch aus seiner Lösung leicht wieder auskristallisiert.

Erfindungsgemäß ist daher auch ein Verfahren zur Stabilisierung von Lösungen des 4,4',4''-(1,3,5-Triazin-2,4,6-triyltriimino)-tris-benzoesäure-tris(2-ethylhexylester) in
(a) entweder einer isolierten Ölkomponente oder
(b) in mindestens einer Ölphase eines dispersen Zwei- oder Mehrphasensystems, welches zusätzlich eine oder mehrere Wasserphasen enthalten kann,
dadurch gekennzeichnet, daß
(a) entweder einer isolierten Ölkomponente, in welcher 4,4',4''-(1,3,5-Triazin-2,4,6-triyltriimino)-tris-benzoesäure-tris(2-ethylhexylester) gelöst vorliegt, oder
(b) mindestens einer Ölphase eines dispersen Zwei- oder Mehrphasensystems in welcher 4,4',4''-(1,3,5-Triazin-2,4,6-triyltriimino)-tris-benzoesäure-tris-(2-ethylhexylester) gelöst vorliegt, wobei das Zwei- oder Mehrphasensystem welches zusätzlich eine oder mehrere Wasserphasen enthalten kann,
ein wirksamer Gehalt an einem oder mehreren erfindungsgemäß verwendeten Campherderivate zugesetzt wird.

Die Gesamtmenge an 4,4',4''-(1,3,5-Triazin-2,4,6-triyltriimino)-tris-benzoesäure-tris(2-ethylhexylester) in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an einem oder mehreren erfindungsgemäß verwendeten Campherderivaten in den fertigen kosmetischen oder dermatologischen Zubereitungen wird vorteilhaft aus dem Bereich von 0,1 - 15,0 Gew.-%, bevorzugt 0,5 - 8,0 Gew.-% gewählt, bezogen auf das Gesamtgewicht der Zubereitungen.

Es ist erfindungsgemäß besonders vorteilhaft, die Gewichtsverhältnisse zwischen 4,4',4''-(1,3,5-Triazin-2,4,6-triyltriimino)-tris-benzoesäure-tris(2-ethylhexylester) und einem oder mehreren erfindungsgemäß verwendeten Campherderivaten aus dem Bereich von 1 : 8 bis 4 : 1, bevorzugt 1 : 4 bis 2 : 1, insbesondere bevorzugt 1 : 2 bis 1 : 1, zu wählen.

Erfindungsgemäße kosmetische und dermatologische Zubereitungen enthalten vorteilhaft außerdem anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden. Besonders bevorzugt handelt es sich um Pigmente auf der Basis von TiO₂.

Es ist besonders vorteilhaft im Sinne der vorliegenden Erfindung, wenngleich nicht zwingend, wenn die anorganischen Pigmente in hydrophober Form vorliegen, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Rektion gemäß

n TiO₂ + m (RO)₃ Si-R' → n TiO₂ (oberfl.)

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte TiO₂-Pigmente sind beispielsweise unter den Handelsbezeichnungen MT 100 T von der Firma TAYCA, ferner M 160 von der Firma Kemira sowie T 805 von der Firma Degussa erhältlich.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zukker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:
- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkybenzoat und 2-Ethylhexylisostearat, Mischungen aus C₁₂₋₁₅-Alkybenzoat und Isotridecylisononanoat sowie Mischungen aus C₁₂₋₁₅-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolrnonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die kosmetischen oder dermatologischen Lichtschutzzubereitungen enthalten vorteilhaft anorganische Pigmente, insbesondere Mikropigmente, z.B. in Mengen von 0,1 Gew.-% bis 30 Gew.-%, vorzugsweise in Mengen von 0,5 Gew.-% bis 10 Gew.-%, insbesondere aber 1 Gew.-% bis 6 Gew.-%, bezogen auf das Gesamtgewicht der Zubereitungen.

Es ist erfindungsgemäß vorteilhaft, außer den erfindungsgemäßen Kombinationen öllösliche UVA-Filter und/oder UVB-Filter in der Lipidphase und/oder wasserlösliche UVA-Filter und/oder UVB-Filter in der wäßrigen Phase einzusetzen.

Vorteilhaft können die erfindungsgemäßen Lichtschutzformulierungen weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die weiteren UVB-Filter können öllöslich oder wasserlöslich sein. Vorteilhafte öllösliche UVB-Filtersubstanzen sind z.B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;

Vorteilhafte wasserlösliche UVB-Filtersubstanzen sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und deren Salze;
- Sulfonsäure-Derivate des 3-Benzylidencamphers, wie z.B. 4-(2-Oxo-3-bornylidenmethyl)benzolsulfonsäure,2-Methyl-5-(2-oxo-3-bornylidenmethyl)sulfonsäure und deren Salze.

Die Liste der genannten weiteren UVB-Filter, die in Kombination mit den erfindungsgemäßen Wirkstoffkombinationen verwendet werden können, soll selbstverständlich nicht limitierend sein.

Es kann auch von Vorteil sein, die erfindungsgemäßen Kombinationen mit weiteren UVA-Filtem zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Ferner ist vorteilhaft, die erfindungsgemäßen Wirkstoffkombinationen mit weiteren UVA- und/oder UVB-Filtern zu kombinieren.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Beispiel 1** Sonnencrème, O/W | |
|---|---|
| | Gew.-% |
| Cyclomethicon | 3,00 |
| Glycerylstearat + PEG-30 Stearat | 2,00 |
| Wollwachsalkohol | 0,10 |
| Glycerylstearat | 3,00 |
| Isopropylpalmitat | 2,00 |
| Octyldodecanol | 1,00 |
| C₁₂₋₁₅-Alkylbenzoat | 2,00 |
| Glycerin | 3,00 |
| Cetylalkohol | 3,00 |
| Myristylmyristat | 2,00 |
| Methylbenzylidencampher | 2,00 |
| Octylmethoxycinnamat | 4,50 |
| Butylmethoxydibenzoylmethan | 2,00 |
| Uvinul T 150 | 2,00 |
| Tocopherylacetat | 0,50 |
| EDTA-Lösung (20 %-ig) | 0,50 |
| Ethylalkohol | 4,00 |
| Konservierungsmittel | q.s. |
| Parfüm | q.s. |
| Wasser, VES | ad 100,00 |

| **Beispiel 2** Sonnencrème, O/W | |
|---|---|
| | Gew.-% |
| Cyclomethicon | 3,00 |
| Glycerylstearat + PEG-30 Stearat | 2,00 |
| Glycerylstearat | 3,00 |
| Isopropylpalmitat | 2,00 |
| Octyldodecanol | 3,00 |
| Glycerin | 3,00 |
| Cetylalkohol | 3,00 |
| Tocopherylacetat | 0,50 |
| Octylmethoxycinnamat | 4,50 |
| Methylbenzylidencampher | 0,50 |
| Uvinul T150 | 1,50 |
| PVP/Hexadecen Copolymer | 1,50 |
| EDTA-Lösung (20 %-ig) | 0,50 |
| NaOH | q.s. |
| Ethylalkohol | 1,50 |
| Konservierungsmittel | q.s. |
| Parfüm | q.s. |
| Wasser, VES | ad 100,00 |

| **Beispiel 3** Sonnenmilch, O/W | |
|---|---|
| | Gew.-% |
| Trilaureth-4 Phosphat | 0,75 |
| Triceteareth-4 Phosphat | 1,00 |
| Glycerylstearat + PEG-100 Stearat | 1,00 |
| Glycerylstearat + Ceteareth-20 | 0,80 |
| Glyceryllanolat | 0,50 |
| Isopropylpalmitat | 3,00 |
| Glycerin-Caprylat/Caproat ("Caprylic/Capric Triglyceride") | 5,00 |
| Cetylalkohol | 1,00 |
| Uvinul T 150 | 1,50 |
| Octylmethoxycinnamat | 6,00 |
| Methylbenzylidencampher | 3,00 |
| PVP/Eicosen Copolymer | 1,00 |
| Butylhydroxytoluol | 0,06 |
| EDTA-Lösung (20 %-ig) | 0,50 |
| NaOH | q.s. |
| Konservierungsmittel | q.s. |
| Wasser, VES | ad 100,00 |

| **Beispiel 4** | |
|---|---|
| | Gew.-% |
| Glycerylstearat SE | 4,00 |
| Stearinsäure | 2,00 |
| C₁₂-C₁₅ Alkyl Benzoate | 5,00 |
| Capric/Caprylic Triglyceride | 5,00 |
| Octyldodecanol | 5,00 |
| Butylenglykol | 5,00 |
| Cetearyl Alkohol | 0,50 |
| Carbomer | 0,20 |
| Butylmethoxydibenzoyl-methan | 2,00 |
| Methylbenzylidencampher | 4,00 |
| Uvinul T150 | 1,00 |
| Tocopherylacetat | 1,00 |
| Furfurylidensorbitol | 0,50 |
| Cyclomethicon | 2,00 |
| Na₃HEDTA | 1,00 |
| Natriumhydroxid (45%ig) | 0.25 |
| Konservierungsmittel | q.s. |
| Parfum | q.s. |
| Wasser, demin. | ad 100,00 |

| **Beispiel 5** | |
|---|---|
| | Gew.-% |
| Glycerylstearat SE | 4,00 |
| Stearinsäure | 2,00 |
| C₁₂-C₁₅ Alkyl Benzoate | 5,00 |
| Capric/Caprylic Triglyceride | 5,00 |
| Octyldodecanol | 5,00 |
| Glycerin | 5,00 |
| Cetearyl Alkohol | 0,50 |
| Carbomer | 0,20 |
| Butylmethoxydibenzoyl-methan | 2,00 |
| Methylbenzylidencampher | 4,00 |
| Uvinul T150 | 1,50 |
| Tocopherylacetat | 1,00 |
| Furfurylidensorbitol | 0,50 |
| Cyclomethicon | 2,00 |
| Na₃HEDTA | 1,00 |
| Natriumhydroxid (45%ig) | 0.25 |
| Konservierungsmittel | q.s. |
| Parfum | q.s. |
| Wasser, demin. | ad 100.00 |

| **Beispiel 6** | |
|---|---|
| | Gew.-% |
| Glycerylstearat SE | 4,00 |
| Stearinsäure | 2,00 |
| C₁₂-C₁₅ Alkyl Benzoate | 5,00 |
| Capric/Caprylic Triglyceride | 5,00 |
| Octyldodecanol | 5,00 |
| Butylenglykol | 5,00 |
| Cetearyl Alkohol | 0,50 |
| Carbomer | 0,20 |
| Butylmethoxydibenzoyl-methan | 2,00 |
| Methylbenzylidencampher | 4,00 |
| Uvinul T150 | 1,50 |
| Tocopherylacetat | 1,00 |
| Furfurylidensorbitol | 0,50 |
| Cyclomethicon | 2,00 |
| Na₃HEDTA | 1,00 |
| Natriumhydroxid (45%ig) | 0.25 |
| Konservierungsmittel | q.s. |
| Parfum | q.s. |
| Wasser, demin. | ad 100.00 |

| **Beispiel 7** | |
|---|---|
| | Gew.-% |
| Glycerylstearat SE | 4,00 |
| Stearinsäure | 2,00 |
| C₁₂-C₁₅ Alkyl Benzoate | 5,00 |
| Mineralöl | 5,00 |
| Octyldodecanol | 5,00 |
| Glycerin | 5,00 |
| Cetearyl Alkohol | 0,50 |
| Carbomer | 0,20 |
| Butylmethoxydibenzoyl-methan | 2,00 |
| Methylbenzylidencampher | 4,00 |
| Uvinul T150 | 1,50 |
| Tocopherylacetat | 1,00 |
| Furfurylidensorbitol | 0,50 |
| Cyclomethicon | 2,00 |
| Na₃HEDTA | 1,00 |
| Natriumhydroxid (45%ig) | 0.25 |
| Konservierungsmittel | q.s. |
| Parfum | q.s. |
| Wasser, demin. | ad 100.00 |

| **Beispiel 8** | |
|---|---|
| | Gew.-% |
| Glycerylstearat SE | 4,00 |
| Stearinsäure | 2,00 |
| C₁₂-C₁₅ Alkyl Benzoate | 5,00 |
| Capric/Caprylic Triglyceride | 5,00 |
| Octyldodecanol | 5,00 |
| Butylenglykol | 5,00 |
| Cetearyl Alkohol | 0,50 |
| Carbomer | 0,20 |
| Butylmethoxydibenzoyl-methan | 2,00 |
| Methylbenzylidencampher | 4,00 |
| Uvinul T150 | 2,00 |
| Tocopherylacetat | 1,00 |
| Furfurylidensorbitol | 0,50 |
| Cyclomethicon | 2,00 |
| Na₃HEDTA | 1,00 |
| Natriumhydroxid (45%ig) | 0.25 |
| Konservierungsmittel | q.s. |
| Parfum | q.s. |
| Wasser, demin. | ad 100,00 |

| **Beispiel 9** | |
|---|---|
| | Gew.-% |
| Glycerylstearat SE | 4,00 |
| Stearinsäure | 2,00 |
| C₁₂-C₁₅ Alkyl Benzoate | 5,00 |
| Capric/Caprylic Triglyceride | 5,00 |
| Octyldodecanol | 5,00 |
| Butylenglykol | 5,00 |
| Cetearyl Alkohol | 0,50 |
| Carbomer | 0,20 |
| Butylmethoxydibenzoyl-methan | 2,00 |
| Methylbenzylidencampher | 4,00 |
| Uvinul T150 | 2,00 |
| Tocopherylacetat | 1,00 |
| Furfurylidensorbitol | 0,50 |
| Cyclomethicon | 2,00 |
| Na₃HEDTA | 1,00 |
| Natriumhydroxid (45%ig) | 0.25 |
| Konservierungsmittel | q.s. |
| Parfum | q.s. |
| Wasser, demin. | ad 100,00 |

## Patentansprüche

1. Verwendung von Campherderivaten der allgemeinen Struktur 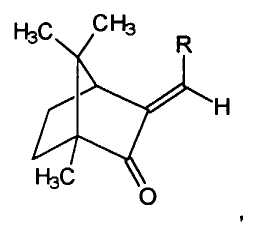 wobei R einen mit bis zu fünf Alkylgruppen substituierten oder unsubstituierten Arylrest darstellt, zur Erzielung oder der Erhöhung der Löslichkeit von 4,4',4''-(1,3,5-Triazin-2,4,6-triyltriimino)-tris-benzoesäure-tris(2-ethylhexylester) in
(a) entweder einer isolierten Ölkomponente oder
(b) in mindestens einer Ölphase eines dispersen Zwei- oder Mehrphasensystem welches zusätzlich eine oder mehrere Wasserphasen enthalten kann.

2. Verfahren zur Stabilisierung von Lösungen des 4,4',4''-(1,3,5-Triazin-2,4,6-triyltriimino)-tris-benzoesäure-tris(2-ethylhexylester) in
(a) entweder einer isolierten Ölkomponente oder
(b) in mindestens einer Ölphase eines dispersen Zwei- oder Mehrphasensystems, welches zusätzlich eine oder mehrere Wasserphasen enthalten kann,
dadurch gekennzeichnet, daß
(a) entweder einer isolierten Ölkomponente, in welcher 4,4',4''-(1,3,5-Triazin-2,4,6-triyltriimino)-tris-benzoesäure-tris(2-ethylhexylester) gelöst vorliegt, oder
(b) mindestens einer Ölphase eines dispersen Zwei- oder Mehrphasensystems in welcher 4,4',4''-(1,3,5-Triazin-2,4,6-triyltriimino)-tris-benzoesäure-tris(2-ethylhexylester) gelöst vorliegt, wobei das Zwei- oder Mehrphasensystem welches zusätzlich eine oder mehrere Wasserphasen enthalten kann,
ein wirksamer Gehalt an einem oder mehreren erfindungsgemäß verwendeten Campherderivate zugesetzt wird.

3. Verwendung nach Anspruch 1 oder Verfahren nach Anspruch 2, dadurch gekennzeichnet, daß die Gesamtmenge an 4,4',4''-(1,3,5-Triazin-2,4,6-triyltriimino)-tris-benzoesäure-tris(2-ethylhexylester) in den fertigen kosmetischen oder dermatologischen Zubereitungen aus dem Bereich von 0,1 - 10,0 Gew.-%, bevorzugt 0,5 - 6,0 Gew.-% gewählt wird, jeweils bezogen auf das Gesamtgewicht der Zubereitungen.

4. Verwendung nach Anspruch 1 oder Verfahren nach Anspruch 2, dadurch gekennzeichnet, daß die Gesamtmenge an einem oder mehreren erfindungsgemäß verwendeten Campherderivaten in den fertigen kosmetischen oder dermatologischen Zubereitungen aus dem Bereich von 0,1 - 15,0 Gew.-%, bevorzugt 0,5 - 8,0 Gew.-% gewählt wird, jeweils bezogen auf das Gesamtgewicht der Zubereitungen.

5. Verwendung nach Anspruch 1 oder Verfahren nach Anspruch 2, dadurch gekennzeichnet, daß die Gewichtsverhältnisse zwischen 4,4',4''-(1,3,5-Triazin-2,4,6-triyltriimino)-tris-benzoesäure-tris(2-ethylhexylester) und einem oder mehreren Campherderivaten aus dem Bereich von 1 : 8 bis 4 : 1, bevorzugt 1 : 4 bis 2 : 1, insbesondere bevorzugt 1 : 2 bis 1 : 1, gewählt wird.

6. Verwendung nach Anspruch 1 oder Verfahren nach Anspruch 2, dadurch gekennzeichnet, daß das disperse Zwei- oder Mehrphasensystem welches zusätzlich eine oder mehrere Wasserphasen enthalten können in Form kosmetischer oder dermatologischer Emulsionen, beispielsweise vom Typ W/O, O/W, W/O/W oder O/W/O vorliegt
